# FASCICULE DE BREVET EUROPEEN

(11) **EP 0 753 033 B1**
(45) Date de publication et mention de la délivrance du brevet: **11.09.2002**
(21) Numéro de dépôt: 95936614.7
(22) Date de dépôt: 27.10.1995
(51) Int. Cl.: C09J 189/06, A61L 26/00

(54) **COLLE CHIRURGICALE, PROCEDE DE PREPARATION ET DISPOSITIF D'APPLICATION ET DE DURCISSEMENT POUR CETTE COLLE**
CHIRURGISCHER KLEBSTOFF, VERFAHREN ZU DEREN HERSTELLUNG, VORRICHTUNG ZUR ANWENDUNG UND HÄRTEN DIESES KLEBSTOFFES
SURGICAL ADHESIVE, METHOD FOR PREPARING SAME AND DEVICE FOR APPLYING IT TOGETHER WITH A HARDENING AGENT

(30) Priorité: 03.11.1994 FR 9413136
(43) Date de publication de la demande: 15.01.1997
(73) Titulaire: FUSION MEDICAL TECHNOLOGIES, INC., Mountain View, CA 94043 (US)
(72) Inventeur: Izoret, Georges, 92310 Sevres (FR)
(74) Mandataire: Orès, Bernard
(86) Numéro de dépôt international: FR9501420
(87) Numéro de publication internationale: WO96014368

(56) Documents cités:
- WO-A-93/04711
- FR-A- 620 957
- US-A- 4 187 119
- DATABASE WPI Week 9415 Derwent Publications Ltd., London, GB; AN 123366 & JP,A,06 070 972 (NIPPON BXI KK) , 15 Mars 1994 & PATENT ABSTRACTS OF JAPAN vol. 18 no. 316 (C-1213) ,16 Juin 1994

## Description

La présente invention concerne des colles chirurgicale à base de gélatine et d'un durcisseur aldéhydique, un procédé pour préparer les colles biologiques et un dispositif pour leur application, ainsi que des durcisseurs pour de telles colles.

Dans le domaine de la chirurgie humaine et animale, vasculaire notamment, il est connu d'utiliser des colles à base de gélatine et de résorcine durcies par un aldéhyde, connues sous le nom de colles GRA. Plus précisément, l'usage de la colle chirurgicale GRF (gélatine résorcine formol) a été souvent décrit pour coller des tissus (cf RBM-volume 4, n°2 - mars/avril 1982, page 147; la Nouvelle Presse Médicale, le 8 mars 1975, 4, n°10).

La gélatine a été choisie notamment en raison de son innocuité et de ses propriétés hémostatiques et par ailleurs pour son aptitude à réagir avec certains aldéhydes dits "durcisseurs" pour former, par réticulation, une masse collante. La résorcine, par ailleurs, permet de renforcer la qualité de ce type de colle.

On connaît l'utilisation de certains aldéhydes tels que le formol officinal à 37%, le glutaraldéhyde à 25% ou encore le mélange de ces deux derniers aldéhydes (9/1), mais on n'utilise ces aldéhydes qu'en solution non gélifiée. Ces aldéhydes présentent l'inconvénient de leur toxicité à de telles concentrations. (DL₅₀ = 296 mg/kg pour le formaldéhyde, DL₅₀ = 33,7 mg/kg pour le glutaraldéhyde).

La mise en oeuvre de telles colles GRF nécessite de la part du chirurgien d'étendre le mélange gélatine/résorcine (GR) sur les surfaces sèches de l'organe à coller, puis à pulvériser, instiller ou déposer par petites gouttes, à l'aide d'une seringue du type seringue intradermique, le durcisseur liquide, solution aqueuse stabilisée de formaldéhyde, sur le mélange GR étalé, et enfin appliquer les surfaces encollées l'une sur l'autre (dossier du CNIMH, novembre-décembre 1984, V.6). Comme durcisseur liquide, il est connu d'utiliser des solutions aqueuses de formaldéhyde, de glutaraldéhyde, de glycéraldéhyde, ou de leurs mélanges.

Cette technique exige une grande précision de la part du chirurgien, du fait notamment de l'action nécrosante du formol. En effet, l'aspersion de formol sur des tissus vivants non concernés par le traitement collant, provoque des dommages et des nécroses souvent irréversibles. La technique chirurgicale consiste jusqu'à présent à protéger très soigneusement les tissus avoisinants, mais ces précautions s'avèrent souvent insuffisantes. Par ailleurs, le risque pour le chirurgien de se piquer avec une séringue remplie de formol n'est jamais nul et, même à de très faibles doses, une injection de formol provoque un choc chez le praticien qui peut alors être obligé d'interrompre son intervention.

Les demandeurs ont maintenant mis au point une colle chirurgicale qui remédie aux inconvénients majeurs des colles connues et présentés ci-dessus.

Ainsi, la présente invention concerne une colle chirurgicale comportant au moins deux constituants destinés à être combinés,
1/ un constituant (A) semi-liquide, comportant au moins de la gélatine en solution aqueuse, et
2/ un constituant (B) gélifié comportant au moins un aldéhyde et au moins un gélifiant biologiquement acceptable qui ne réagit pas avec l'aldéhyde.

Aussi, selon l'invention, le constituant (B) est gélifié et les inconvénients présentés ci-dessus sont évités car les gels durcisseurs sont plus faciles à utiliser que les liquides durcisseurs et ils évitent les risques d'aspersion involontaires et par conséquent les nécroses et ne nécessitent pas l'emploi des mêmes seringues. Ces colles s'appliquent au collage de tissus humains ou animaux et notamment au collage des artères et des aortes.

Le premier constituant (A) est usuel dans les colles GRA : il s'agit d'une solution aqueuse de gélatine, sous forme semi-liquide. Elle peut comporter de 5 à 20 g de gélatine, pour 20 à 25 ml d'eau distillée. Usuellement, le constituant semi-liquide GR comporte environ 15 g de gélatine pour 20 ml d'eau distillée.

La solution aqueuse de gélatine peut en outre contenir, en mélange avec la gélatine, de la résorcine ou du phloroglucinol. Usuellement, on peut utiliser environ 4 à 6 g de résorcine pour 20 à 25 ml d'eau, et de préférence, elle peut en comporter environ 5 g pour environ 15 g de gélatine et environ 20 ml d'eau. La toxicité de la résorcine n'étant pas nulle, il peut être utile de disposer d'une colle n'en comportant que peu ou pas du tout.

Selon un mode préféré de réalisation de l'invention, le constituant (B) comportant l'aldéhyde durcisseur se présente sous forme d'un gel aqueux. Parmi les gélifiants utilisables, on peut citer tous les gélifiants disponibles, usuels, biologiquement acceptables et qui ne réagissent pas avec les aldéhydes. Parmi les gélifiants utilisables, on peut citer notamment l'agar-agar, la gomme de Karaya, les poudres de silice, les dérivés hydroxylés et carboxylés de la cellulose, tels que l'hydroxyéthylcellulose, la carboxyméthylcellulose et leurs mélanges.

Selon l'invention, les gélifiants préférés sont l'hydroxyéthylcellulose et la carboxyméthylcellulose. Ces gélifiants sont non toxiques.

Ces gélifiants sont utilisés à raison de 0,5 à 4% en poids, de préférence environ 1,5 à 2% en poids dans le gel aqueux.

Selon l'invention, les aldéhydes utilisables sont choisis parmi le formaldéhyde officinal, le glutaraldéhyde, le glycéraldéhyde, le dialdéhydc succinique, le sulfonyl-bis-acétaldéhyde, l'octanedial-1.8, l'octène-4 dial-1,8, l'oxy-bis-acétaldéhyde, ainsi que les mélanges de ces aldéhydes.

Dans le second constituant (B) gélifié, les aldéhydes usuels sont utilisés dans leurs concentrations usuelles pour les durcisseurs liquides, éventuellement en mélange, comme dans les durcisseurs liquides. Parmi les aldéhydes, on préfère le dialdéhydc succinique dont l'utilisation comme durcisseur pour colles biologiques n'est pas encore décrite. Cet aldéhyde est efficace à des concentrations très basses, de l'ordre de 5% en poids dans le durcisseur, ce qui présente un avantage eu égard à la toxicité des aldéhydes.

Parmi les mélanges, l'on préfère les mélanges dialdéhyde succinique/glutaraldéhyde (5/0.25).

Les concentrations usuelles en aldéhyde dans les durcisseurs liquides peuvent atteindre des valeurs de l'ordre de 1 à 50% en poids en aldéhydes et, à titre indicatif, 1 à 40% en poids pour le glutaraldéhyde, 1 à 50% en poids pour l'oxy-bis-acétaldéhyde.

Pour le dialdéhyde succinique, on prévoit des concentrations pouvant aller de 1 à 40%, de préférence d'environ 5 à 20%.

Pour les mélanges, on peut citer le mélange usuel formol/glutaraldéhyde (9 parties en volume de formol à 37% pour 1 partie de glutaraldéhyde à 40%) et le mélange de dialdéhyde succinique comportant, en poids dans le constituant (B), de 40 à 50% de dialdéhyde succinique et de 0,2 à 1% de glutaraldéhyde.

L'agent gélifiant est éventuellement utilisé, en solution aqueuse, dans des proportions de l'ordre de 1 à 5% en poids, de préférence 2% en poids, cette proportion dépendant, d'une part, du gélifiant utilisé et, d'autre part, de la viscosité du constituant (A) semi-liquide. En effet, lors de l'application, il peut être utile que les constituants (A) et (B) aient des viscosité similaires.

L'invention concerne également des solutions aqueuses gélifiées de ces aldéhydes ou leurs mélanges, stabilisées ou non, utiles notamment à titre de durcisseur pour colles biologiques.

Les gels d'aldéhyde obtenus avec ces produits sont stables à la température ambiante pendant au moins 1 an. Par ailleurs, la teneur initiale en aldéhyde se maintient pendant cette période.

L'invention concerne également l'utilisation du dialdéhyde succinique à titre de durcisseur de colles chirurgicales. Les colles biologiques qui peuvent être durcies par un tel dialdéhyde succinique, en solution aqueuse gélifiée, comportent, d'une part, un constituant semi-liquide comportant au moins de la gélarine. et éventuellement de la résorcine ou du phloroglucinol et, d'autre parts un constituant aldéhydique, sous forme gélifiée, comportant, à titre de durcisseur, au moins du dialdéhyde succinique. Le dialdéhyde peut être éventuellement mélangé avec un autre aldéhyde durcisseur. Compte tenu de sa grande efficacité, ce dialdéhyde est particulièrement adapté au collage du foie, du poumon, de la rate, du péricarde et du rein. Son efficacité permet de réduire le pourcentage d'aldéhyde utilisé, et ce jusqu'à environ 5 % en poids d'aldéhyde dans le constituant (B).

Dans ces colles chirurgicales au dialdéhyde succinique, de dialdéhyde succinique, dans le constituant (B) gélifié, est utilisé à des concentrations allant d'environ 1 à 40 %, de préférence 5 à 20 % en poids.

La présente invention concerne également un procédé de préparation d'une colle chirurgicale, caractérisé en ce que l'on mélange ou l'on applique en couches superposées, d'une part, un constituant (A) semi-liquide comportant au moins de la gélatine en solution aqueuse et, d'autre part, un constituant (B) gélifié comportant au moins un aldéhyde et au moins un gélifiant biologiquement acceptable qui ne réagit pas avec l'aldéhyde.

De préférence, on mélange les deux constituants juste avant l'emploi.

Pour la préparation de la colle, on procède en préparant, d'une part, le constituant semi-liquide (A) en diluant la gélatine, éventuellement la résorcine, dans de l'eau distillée et, d'autre part, en introduisant l'aldéhyde et le gélifiant de l'eau.

Lors de l'utilisation, on préchauffe usuellement le constituant (A) à la température de l'ordre de 28 à 40°, au bain-marie, de préférence à une température de l'ordre de 37°.

Selon l'invention, les gels d'aldéhyde sont utilisés à température ambiante, mais peuvent être utilisés à des températures de l'ordre de la température humaine, c'est-à-dire des températures de l'ordre de 28 à 40°.

L'utilisation des durcisseurs sous forme de gel permet l'emploi d'un dispositif du type seringue à deux corps, ou tout autre moyen approprié pour mélanger et doser, en proportion convenable, le constituant (A) et le constituant (B) comportant l'aldéhyde choisi à la concentration la plus adéquate au type du collage envisagé.

Cette façon d'opérer libère le chirurgien de l'obligation de pratiquer lui-même le mélange extemporanément ou d'appliquer la colle en deux temps. Il en résulte non seulement un gain de temps mais encore un meilleur dosage des constituants et enfin l'annulation du risque consistant à appliquer, par erreur, le durcisseur sur les tissus voisins des tissus à coller.

L'invention concerne donc un dispositif à deux compartiments dont l'un comporte le constituant (A) et l'autre le constituant (B), tels que définis ci-dessus.

Un dispositif convenable est du type seringue à deux corps comportant, dans l'un de ses corps, un constituant (A) comportant au moins de la gélatine en solution aqueuse et, dans l'autre corps, le constituant (B) gélifié selon la présente invention, comportant au moins un aldéhyde et au moins un gélifiant biologiquement acceptable qui ne réagit pas avec l'aldéhyde.

Dans un mode particulier de réalisation de l'invention, on dispose d'une seringue à deux corps qui permet l'application simultanée des deux composants, ou le mélange des deux composants juste à la sortie de la seringue.

Des seringues bi-corps de ce type sont notamment connues sous la dénomination commerciale « Duploject ».

De préférence, et notamment dans ce mode d'application, on utilise des constituants (A) et (B) de viscosité similaire.

Les colles chirurgicales préparées selon l'invention se sont montrées particulièrement efficaces, notamment pour le collage aortique, celui du péricarde, du poumon, du foie, de la rate ou du rein. De façon générale, les colles de l'invention peuvent être utilisées en chirurgie osseuse, vasculaire, viscérale et dentaire.

Les exemples ci-dessous permettent d'illustrer l'invention sans en limiter la portée.

### EXEMPLE 1

### CONSTITUANT (A)

Une pâte GR est obtenue par dissolution, dans 20 à 25 ml d'eau distillée, maintenue au bain-marie à 50°C, de 20 g d'un mélange de résorcine et de gélatine en poudre dans des proportions respectives de 1/4 et 3/4. La consistance convenable du mélange est obtenue en 30 minutes grâce au mixage par un agitateur du type planétaire manipulé lentement pour éviter la formation de bulles d'air.

### CONSTITUANT (B)

A 9 volumes d'une solution aqueuse de formol à 37% en poids, on ajoute un volume d'une solution aqueuse de glutaraldéhyde à 25% en poids, puis 1,5 à 2% en poids d'hydroxyéthylcellulose. On chauffe le mélange, sous agitation mécanique, au bain-marie jusqu'à dissolution complète de l'hydroxyéthylcellulose. Après refroidissement à la température ambiante, on obtient un gel de viscosité similaire à celle du mélange GR. La viscosité obtenue est fonction de la quantité d'hydroxyéthylcellulose mise en oeuvre et le pourcentage indiqué dans cet exemple n'est pas limitatif.

On applique le mélange des constituants (A) et (B) sur les tissus à coller et on applique ces tissus l'un sur l'autre.

### EXEMPLE 2

### CONSTITUANT (A)

On utilise une pâte GR préconditionnée, provenant du mélange gélatine-résorcine-eau illustré à l'exemple 1, dans un tube stérile prêt à l'emploi, après chauffage pendant 5 à 10 minutes, dans un bain-marie stérile à 45°C.

### CONSTITUANT (B)

A une solution aqueuse contenant 5% en poids de dialdéhyde succinique et 0,25% en poids de glutaraldéhyde, on ajoute 2% en poids de carboxyméthylcellulose. On chauffe et agite comme dans l'exemple 1 pour obtenir un gel (B).

On applique le mélange des constituants (A) et (B) sur les tissus à coller et on applique ces tissus l'un sur l'autre.

### EXEMPLE 3

On reproduit l'exemple 2, mais avec une solution aqueuse à 5% de dialdéhyde succinique, et sans glutaraldéhyde, à titre de constituant, (B).

On applique le mélange des constituants (A) et (B) sur les tissus à coller et on applique ces tissus l'un sur l'autre.

### EXEMPLE 4

Le constituant (A) est identique à celui de l'exemple 1.

On prépare un gel aqueux contenant 36% en poids de formaldéhyde et 4,5% en poids de dialdéhyde succinique et 2% en poids de carboxyméthylcellulose. On chauffe et agite comme dans l'exemple 1, pour obtenir le gel désiré (B).

On applique le mélange des constituants (A) et (B) sur les tissus à coller et on applique ces tissus l'un sur l'autre.

## Revendications

1. Colle chirurgicale comportant au moins deux constituants destinés à être combinés, **caractérisée en ce qu'**elle comporte:
1/ un constituant (A) semi-liquide, comportant au moins de la gélatine en solution aqueuse, et
2/ un constituant (B) gélifié, comportant au moins un aldéhyde et au moins un gélifiant biologiquement acceptable qui ne réagit pas avec l'aldéhyde.

2. Colle selon la revendication 1, **caractérisée en ce que** le constituant (A) comporte de 5 à 20 g de gélatine pour 20 à 25 ml d'eau, de préférence 15 g de gélatine pour 20 ml d'eau et comporte en outre de 4 à 6 g de résorcine ou de phloroglucinol pour 20 à 25 ml d'eau, de préférence 5 g de résorcine pour 20 ml d'eau.

3. Colle selon la revendication 1 ou 2, **caractérisée en ce que** le constituant (B) comporte de 0,5 à 4 % en poids, de préférence 1,5 à 2 % en poids d'un gélifiant choisi parmi l'agar-agar, la gomme de Karaya, les poudres de silice, l'hydroxyéthylcellulose, la carboxyméthylcellulose, les dérivés hydroxylés et carboxylés de la cellulose, et leurs mélanges.

4. Colle selon l'une des revendications 1 à 3, **caractérisée en ce que** le constituant (B) comporte de 1 à 50 % d'aldéhyde.

5. Colle selon la revendication 1 à 4, **caractérisée en ce que** l'aldéhyde est choisi parmi le formaldéhyde, le glutaraldéhyde, le glycéraldéhyde, le dialdéhyde succinique, l'oxy-bis-acétaldéhyde, le sulfonyl-bis-acétaldéhyde, l'octanedial-1,8, l'octène-4 dial-1,8, et leurs mélanges.

6. Colle selon l'une des revendications 1 à 5, **caractérisée en ce que** le constituant (B) comporte de 1 à 40% en poids, de préférence 5 à 20 % en poids de dialdéhyde succinique.

7. Utilisation de dialdéhyde succinique en association avec la gélatine ou un mélange de gélatine et de résorcine, dans une colle chirurgicale comportant un constituant (A) semi-liquide comportant au moins de la gélatine en solution aqueuse, et un constituant (B) comportant au moins ledit dialdéhyde succinique en solution aqueuse gélifiée.

8. Utilisation de solutions aqueuses gélifiées, comportant au moins un aldéhyde, capable de durcir des mélanges à base de gélatine à titre de durcisseur pour colles chirurgicale, et au moins un gélifiant biologiquement acceptable qui ne réagit pas avec l'aldéhyde.

9. Dispositif à deux compartiments dont l'un comporte le constituant (A) et l'autre le constituant (B), tels que définis dans l'une des revendications 1 à 8.

10. Procédé de préparation d'une colle chirurgicale, **caractérisé en ce que** l'on mélange ou l'on applique en couches superposées, d'une part, un constituant (A) semi-liquide comportant au moins de la gélatine en solution aqueuse et, d'autre part, un constituant (B) gélifié comportant au moins un aldéhyde et au moins un gélifiant biologiquement acceptable qui ne réagit pas avec l'aldéhyde.

## Patentansprüche

1. Chirchurgischer Klebstoff mit mindestens zwei miteinander zu kombinierenden Bestandteilen, **dadurch gekennzeichnet, daß** er aufweist:
1/ einen halbflüssigen Bestandteil (A), der mindestens Gelatine in wäßriger Lösung umfaßt, und
2/ einen gelierten Bestandteil (B), der mindestens einen Aldehyd und mindestens einen physiologisch unbedenklichen Gelbildner, welcher nicht mit dem Aldehyd reagiert, umfaßt.

2. Klebstoff nach Anspruch 1, **dadurch gekennzeichnet, daß** der Bestandteil (A) 5 bis 20 g Gelatine auf 20 bis 25 ml Wasser, vorzugsweise 15 g Gelatine auf 20 ml Wasser, und des weiteren 4 bis 6 g Resorcin oder Phloroglucinol auf 20 bis 25 ml Wasser, vorzugsweise 5 g Resorcin auf 20 ml Wasser umfaßt.

3. Klebstoff nach Anspruch 1 oder 2, **dadurch gekennzeichnet, daß** der Bestandteil (B) 0,5 bis 4 Gew.-% vorzugsweise 1,5 bis 2 Gew.-% eines unter Agar-Agar, Karaya-Gummi, Pulvern von Kieselsäure, Hydroxyethylcellulose, Carboxymethylcellulose, den Hydroxyl- und Carboxylderivaten von Cellulose, sowie deren Mischungen ausgewählten Gelbildners umfaßt.

4. Klebstoff nach einem der Ansprüche 1 bis 3, **dadurch gekennzeichnet, daß** der Bestandteil (B) 1 bis 50% Aldehyd umfaßt.

5. Klebstoff nach einem der Ansprüche 1 bis 4, **dadurch gekennzeichnet, daß** der Aldehyd unter Formaldehyd, Glutaraldehyd, Glycerinaldehyd, Bernsteinsäuredialdehyd, Oxy-bis-acetaldehyd, Sulfonyl-bis-acetaldehyd, Octan-1,8-dial, Oct-4-en-1,8-dial und deren Mischungen ausgewählt ist.

6. Klebstoff nach einem der Ansprüche 1 bis 5, **dadurch gekennzeichnet, daß** der Bestandteil (B) 1 bis 40 Gew.-%, vorzugsweise 5 bis 20 Gew.-% Bernsteinsäuredialdehyd umfaßt.

7. Verwendung von Bernsteinsäuredialdehyd in Verbindung mit Gelatine oder einer Mischung von Gelatine und Resorcin in einem chirurgischen Klebstoff, welcher einen mindestens Gelatine in wäßriger Lösung umfassenden halbflüssigen Bestandteil (A) und einen mindestens den Bernsteinsäuredialdehyd in gelierter wäßriger Lösung umfassenden Bestandteil (B) aufweist.

8. Verwendung von gelierten wäßrigen Lösungen mit mindestens einem Aldehyd, welcher in der Lage ist, auf Gelatine basierende Mischungen als Aushärtungsmittel für chirurgische Klebstoffe auszuhärten, und mindestens einem physiologisch unbedenklichen Gelbildner, der nicht mit dem Aldehyd reagiert.

9. Vorrichtung mit zwei Fächern, von denen eines den Bestandteil (A) und das andere den Bestandteil (B) gemäß der Definition in einem der Ansprüche 1 bis 8 beinhaltet.

10. Verfahren zur Bereitung eines chirurgischen Klebstoffes, **dadurch gekennzeichnet, daß** zum einen ein halbflüssiger Bestandteil (A), der mindestens Gelatine in wäßriger Lösung umfaßt, und zum anderen ein gelierter Bestandteil (B), der mindestens einen Aldehyd und mindestens einen physiologisch unbedenklichen Gelbildner, der nicht mit dem Aldehyd reagiert, umfaßt, vermischt oder in übereinanderliegenden Schichten appliziert werden.

## Claims

1. Surgical adhesive comprising at least two constituents intended to be combined, **characterised in that** it comprises:
1) a semi-liquid constituent (A) comprising at least gelatin in aqueous solution, and
2) a gelled constituent (B) comprising at least one aldehyde and at least one biologically acceptable gelling agent which does not react with the aldehyde.

2. Adhesive according to claim 1, **characterised in that** constituent (A) comprises from 5 to 20 g of gelatin to 20 to 25 ml of water and preferably 15 g of gelatin to 20 ml of water and also comprises from 4 to 6 g of resorcinol or phloroglucinol to 20 to 25 ml of water and preferably 5 g of resorcinol to 20 ml of water.

3. Adhesive according to claim 1 or 2, **characterised in that** constituent (B) comprises from 0.5 to 4% by weight, and preferably 1.5 to 2% by weight, of a gelling agent selected from agar, Karaya gum, silica powders, hydroxyethylcellulose, carboxymethylcellulose, hydroxylated and carboxylated derivates of cellulose, and mixtures thereof.

4. Adhesive according to one of claims 1 to 3, **characterised in that** constituent (B) comprises from 1 to 50% aldehyde.

5. Adhesive according to claims 1 to 4, **characterised in that** the aldehyde is selected from formaldehyde, glutaraldehyde, glyceraldehyde, succinic dialdehyde, oxy-bis-acetaldehyde, sulphonyl-bis-acetaldehyde, octanedial-1,8, octane-4 dial-1,8, and mixtures thereof.

6. Adhesive according to one of claims 1 to 5, **characterised in that** constituent (B) comprises from 1 to 40% by weight, and preferably 5 to 20% by weight, of succinic dialdehyde.

7. Use of succinic dialdehyde in association with gelatin or a mixture of gelatin and resorcinol, in a surgical adhesive comprising a semi-liquid constituent (A) comprising at least gelatin in aqueous solution, and a constituent (B) comprising at least said succinic dialdehyde in gelled aqueous solution.

8. Use of gelled aqueous solutions, comprising at least one aldehyde capable of hardening gelatin-based mixtures as a hardener for surgical adhesives, and at least one biologically acceptable gelling agent which does not react with the aldehyde.

9. Device having two compartments of which one contains constituent (A) and the other constituent (B), the constituents being as defined in one of claims 1 to 8.

10. Method of preparing a surgical adhesive, **characterised in that** there are mixed, or are applied in superimposed layers, on the one hand a semi-liquid constituent (A) comprising at least gelatin in aqueous solution, and on the other hand a gelled constituent (B) comprising at least one aldehyde and at least one biologically acceptable gelling agent which does not react with the aldehyde.
